# EUROPÄISCHE PATENTANMELDUNG

(11) **EP 1 083 163 A2**
(43) Veröffentlichungstag der Anmeldung: **14.03.2001**
(21) Anmeldenummer: 00118912.5
(22) Anmeldetag: 01.09.2000
(51) Int. Cl.: C07C 209/08, C07C 209/10

(54) **Katalytisches Verfahren zur Herstellung von aromatischen Aminen unter Verwendung von Palladaphosphacyclobutanen**

(30) Priorität: 09.09.1999 DE 19942961
(71) Anmelder: Clariant GmbH, 65929 Frankfurt am Main (DE)
(72) Erfinder: Geissler, Holger, Dr., 55116 Mainz (DE); Haber, Steffen, Dr., 76829 Landau/Pfalz (DE); Scherer, Stefan, Dr., 64572 Büttelborn (DE); Meudt, Andreas, Dr., 64539 Flörsheim-Weilbach (DE); Vollmüller, Frank, Dr., 55130 Mainz (DE)

(57) **Zusammenfassung**

Aromatische Amine der Formel (I)

Ar-[NR⁶R⁷]ₙ (I)

werden hergestellt durch Umsetzung eines Halogenaromaten der Formel (II)

Ar-Hal (II)

mit einem Amin der Formel (III)

R⁶R⁷NH (III)

in Gegenwart eines Palladaphosphacyclobutans und einer Base, gegebenenfalls in Gegenwart eines ionischen Halogenids, in einem Lösungsmittel bei Temperaturen von 20 bis 200°C.

## Beschreibung

Die vorliegende Erfindung betrifft ein neues Verfahren zur Herstellung von aromatischen Aminen mit Palladaphosphacyclobutanen.

Aromatische Amine, insbesondere substituierte Aniline, sind von großer industrieller Bedeutung als Vorprodukte für Farbstoffe, Feinchemikalien, Agroprodukte und Wirkstoffvorprodukte.

Die Herstellung von substituierten Anilinen wird technisch im allgemeinen durch Nitrierung eines entsprechenden Aromaten und nachfolgende Hydrierung durchgeführt. Da Nitrierungen unter drastischen Reaktionsbedingungen stattfinden, sind eine Vielzahl komplex substituierter Aniline nicht oder nur unzureichend auf diesem Wege darstellbar.

Palladiumkatalysierte Aminierungen von Jod-, Brom- und Chloraromten, die zu substituierten Anilinen führen, sind in A.S. Guram et al., Angew. Chem. 1995, 107, 1459, beschrieben. Diese Reaktionen werden unter vergleichsweise milden Reaktionsbedingungen durchgeführt und können daher auch für die Synthese von komplex substituierten Anilinen eingesetzt werden. Die als Ausgangsverbindungen verwendeten Iod- und Bromaromaten sind im Gegensatz zu den Chloraromaten wesentlich teurer und schwerer zugänglich.

Die DE-A1-196 50 213 offenbart ein Verfahren zur Aminierung von Chloraromaten unter Einsatz von trans-Di-µ-acetato-bis-(o-(di-o-tolylphosphino)benzyl)dipalladium, gegebenenfalls in Gegenwart von Halogenid-Cokatalysatoren. In der Regel wird 1 mol% Katalysator (entsprechend 2 mol% Pd) eingesetzt.

Insbesondere bei Chloraromaten werden generell große Mengen an Katalysator, im allgemeinen 1 bis 5 Mol-%, zugesetzt, um technisch nutzbare Umsätze zu erzielen. Aufgrund der Komplexität der Reaktionsgemische ist kein einfaches Katalysatorrecycling möglich, so daß die Katalysatorkosten in der Regel einer technischen Realisierung entgegenstehen.

Daher bestand ein großer Bedarf nach einem Verfahren zur Herstellung aromatischer Amine, das die genannten Nachteile nicht aufweist, für eine technische Durchführung geeignet ist und aromatische Amine in hoher Ausbeute und Reinheit liefert.

Diese Aufgabe wird überraschenderweise durch die Verwendung bestimmter Palladaphosphacyclobutane als Katalysatoren gelöst.

Gegenstand der vorliegenden Erfindung ist ein Verfahren zur Herstellung von aromatischen Aminen der Formel (I)

Ar-[NR⁶R⁷]ₙ (I)

worin n für die Zahl 1, 2 oder 3 steht,
Ar für unsubstituiertes oder substituiertes Phenyl, Furanyl, Pyrryl, Pyridinyl, Naphthyl oder Chinolinyl steht, worin die Substituenten 1, 2, 3, 4, 5 oder 6, vorzugsweise 1, 2 oder 3, an der Zahl und aus der Gruppe C₁-C₈-Alkyl, C₃-C₈-Cycloalkyl, Alkoxy-(C₁-C₈), Acyloxy-(C₁-C₈), C₆-C₁₀-Aryloxy, C₆-C₁₀-Aryl,Benzyl, Fluor, Chlor, Brom, OH, NO₂, OSO₂CF₃, CN, COOH, CHO, SO₃H, SO₂R, SOR, wobei R (C₁-C₄)-Alkyl, (C₆-C₁₀)-Aryl oder Benzyl bedeutet, NH₂, NHAlkyl (C₁-C₈), N-Alkyl₂-(C₁-C₈), CF₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, CHCH-CO₂Alkyl-(C₁-C₈), CHCHCO₂H, PO-Phenyl₂, PO-Alkyl₂-(C₁-C₄), 5-Ring-heteroaryl und 6-Ring-heteroaryl jeweils mit O, S und/oder N als Heteroatome sind; und R⁶ und R⁷ unabhängig voneinander Wasserstoff, (C₁-C₁₂)-Alkyl, Hydroxyalkyl (C₁-C₁₂), unsubstituiertes oder substiutiertes Phenyl, oder C₃-C₈-Cycloalkyl sind, oder R⁶ und R⁷ zusammen mit dem N-Atom einen 5- oder 6-gliedrigen aliphatischen oder aromatischen Ring bilden, der 1 oder 2 weitere Atome aus der Gruppe N, O und S als Heteroatome enthalten kann,
   durch Umsetzung von Halogenaromaten der allgemeinen Formel (II)

   Ar-Hal (II)
worin Hal die Bedeutung Cl, Br oder l besitzt,
   mit einem Amin der allgemeinen Formel (III)

   R⁶R⁷NH (III)

   dadurch gekennzeichnet, daß man die Umsetzung in Gegenwart eines Palladaphosphacyclobutans der allgemeinen Formel (IV) 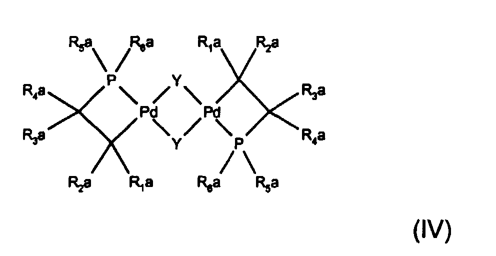 worin
R^{1a}, R^{2a} unabhängig voneinander Wasserstoff, (C₁-C₄)-Alkyl, (C₃-C₁₂)-Cycloalkyl, (C₁-C₄)-Alkoxy, Fluor, N-(C₁-C₄-Alkyl)₂, CO₂-(C₁-C₄-Alkyl), OCO-(C₁-C₄-Alkyl), oder substituiertes oder unsubstituiertes Aryl,
R^{3a}, R^{4a}, R^{5a} und R^{6a} unabhängig voneinander (C₁-C₈)-Alkyl, (C₃-C₁₂)-Cycloalkyl, substituiertes oder unsubstituiertes Aryl;
   oder worin R^{1a} und R^{2a}, oder R^{2a} und R^{3a}, oder R^{3a} und R^{4a} zusammen einen aliphatischen Ring mit 4 bis 10 C-Atomen bilden,
   oder worin R^{5a} und R^{6a} zusammen mit dem P-Atom einen gesättigten oder ungesättigten 4- bis 9-gliedrigen Ring bilden, oder R^{4a} und R^{5a} eine überbrückende 1,ω-Alkandiyl-Kette mit 2 bis 7 C-Atomen bilden, und
Y ein Anion einer anorganischen oder organischen Säure, eine α,γ-Diketoverbindung oder einen 5- bis 6-gliedrigen stickstoffhaltigen Heterocyclus bedeutet, in Gegenwart einer Base, und gegebenenfalls in Gegenwart eines ionischen Halogenides, in einem Lösemittel bei Temperaturen von 20 bis 200°C umsetzt.

Die Synthese der Palladaphosphacyclobutane ist in DE-A1-196 47 584 beschrieben. Bevorzugt sind Verbindungen der Formel (IV), worin
R^{1a} und R^{2a} unabhängig voneinander Wasserstoff, Methyl, Ethyl, Cyclopentyl, Cyclohexyl, Methoxy, Ethoxy, Fluor, Phenyl, Tolyl oder Naphthyl;
R^{3a} und R^{4a} unabhängig voneinander (C₁-C₄)-Alkyl, (C₅-C₆)-Cycloalkyl, substituiertes oder unsubstituiertes C₆-C₁₀-Aryl oder worin R^{3a} und R^{4a} zusammen einen aliphatischen Ring mit 5 bis 6 C-Atomen bilden;
R^{5a} und R^{6a} unabhängig voneinander (C₁-C₄)-Alkyl, (C₅-C₆)-Cycloalkyl, Phenyl, Naphthyl, Anthracenyl, die unsubstituiert oder mit 1 bis 3 CF₃-, (C₁-C₄)-Alkyl oder (C₁-C₄)-Alkoxy Gruppen substituiert sind;
und Y für Acetat, Propionat, Benzoat, Chlorid, Bromid, Iodid, Fluorid, Sulfat, Hydrogensulfat, Nitrat, Phosphat, Trifluoromethansulfonat,Tetrafluoroborat, Tosylat, Mesylat, Acetylacetonat, Hexafluoracetylacetonat oder Pyrazolyl steht, bedeuten.

Besonders bevorzugt sind Verbindungen der Formel (IV), worin
- R^{1a} und R²a: unabhängig voneinander Wasserstoff oder Methyl;
- R^{3a} und R^{4a}: unabhängig voneinander Methyl, Ethyl oder Phenyl;
- R^{5a} und R^{6a}: unabhängig voneinander Phenyl, Naphthyl, o-Trifluoromethylphenyl, o-Trifluoromethyl-p-tolyl, o-Trifluoromethyl-p-methoxy-phenyl, o-Methoxy-phenyl, o,p-Dimethoxy-phenyl, o,o,p-Trimethoxy-phenyl, Anthracenyl, tert.-Butyl, n-Butyl, Isopropyl, Isobutyl, Cyclohexyl oder 1-Methylcyclohexyl bedeuten.

Ganz besonders bevorzugt sind folgende Verbindungen der Formel (IV):
trans-Di-µ-acetato-bis[2-[bis(1,1-dimethylethyl)phosphino]-2-methylpropyl-C,P]dipalladium (II),
trans-Di-µ-acetato-bis[2-[(1,1-dimethylethyl)-phenylphosphino]-2-methylpropyl-C,P]dipalladium (II),
trans-Di-µ-chloro-bis[2-[bis(1,1-dimethylethyl)phosphino]-2-methylpropyl-C,P]dipalladium (II),
trans-Di-µ-chloro-bis[2-[(1,1-dimethylethyl)-phenylphosphino]-2-methylpropyl-C,P]dipalladium (II),
trans-Di-µ-bromo-bis[2-[bis(1,1-dimethylethyl)phosphino]-2-methylpropyl-C,P]dipalladium (II) sowie
trans-Di-µ-bromo-bis[2-[(1,1-dimethylethyl)-phenylphosphino]-2-methylpropyl-C,P]dipalladium (II).

Die Palladiumkatalysatoren werden vor der eigentlichen Reaktion synthetisiert, können jedoch auch in situ erzeugt werden, wie z.B. in EP-A1-0802173 beschrieben. Bei längerer Reaktionsführung erweisen sich die in situ Mischungen (molares Verhältnis Pd:P = 1:1) jedoch als wenig stabil und führen häufig zur Palladiumabscheidung. Dieser Nachteil wird beim erfindungsgemäßen Einsatz bereits fertiggestellter Palladaphosphacyclobutane überraschenderweise überwunden.

Palladaphosphacyclobutane haben in der Regel einen dimeren Aufbau. Bei bestimmten Verbindungen (z.B. Y = Acetylaceton, Hexafluoracetylaceton) können jedoch auch monomere, oligomere oder gar polymere Strukturen vorliegen.

Während des Katalysezyklus wird durch Brückenspaltungsreaktionen mit anorganischen und organischen Nucleophilen die dimere Struktur aufgebrochen, so daß als eigentlich katalytisch aktive Spezies die einkernigen Komplexe der Formel (V) bzw. (VI) 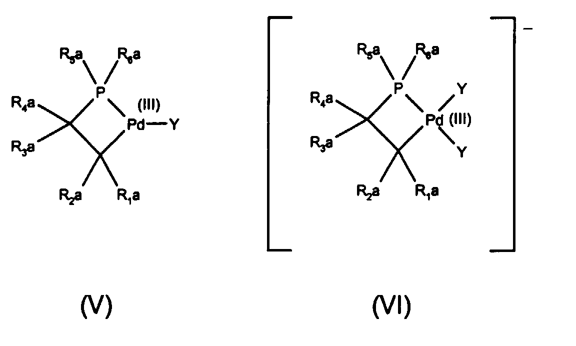 in Betracht zu ziehen sind. Die Komplexe der Formel (V) und (VI) stehen mit den eingesetzten Dimeren im Austauschgleichgewicht und haben neutralen oder anionischen Charakter. Der einkernige Komplex der Formel (V) kann dabei gegebenenfalls weitere Donorliganden am Palladiumatom enthalten.

Die eingesetzten Palladaphosphacyclobutane zeichnen sich durch sehr große Aktivität und überraschend hoher Stabilität aus.

Die Stabilität der Palladaphosphacyclobutane in Lösung läßt sich durch Zusatz von Alkali-, Erdalkali- und Übergangsmetallsalzen der 6. bis 8. Nebengruppe noch weiter erhöhen. Insbesondere der Zusatz von ionischen Halogeniden und Pseudohalogeniden (z.B. CN⁻) bewirken bei der Umsetzung von Chloraromaten signifikante Ausbeutesteigerungen und Standzeitverbesserungen des Homogenkatalysators.

Vorzugsweise ist das ionische Halogenid ein Alkali-, Ammonium-, Alkylammonium-, Alkylolammonium- oder Phosphoniumhalogenid, insbesondere ein Alkali- oder Ammoniumhalogenid, wobei Halogenid die Bedeutung Chlorid, Bromid oder Jodid, insbesondere Bromid oder Chlorid hat. Beispiele sind Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumjodid, Lithiumjodid, Natriumjodid, Kaliumjodid und/oder Tetrabutylphosphoniumjodid, insbesondere Lithiumchlorid, Ammoniumchlorid, Dimethylammoniumchlorid, und/oder Diethanolammoniumchlorid.

Das ionische Halogenid wird vorzugsweise in Mengen von 0,1 bis 100 mol%, insbesondere von 3 bis 50 mol%, bezogen auf den eingesetzten Halogenaromaten eingesetzt. Es kann auch in Form eines flüssigen Salzes als Lösemittel dienen.

Aufgrund der Katalysatoraktivitäten und -stabilität ist es möglich, sehr kleine Mengen an Katalysator zu verwenden, so daß die Katalysatorkosten im Vergleich zu herkömmlichen Verfahren für den entsprechenden Prozeß nicht kostenlimitierend sind.

Der Katalysator kann in Mengen von 0,001 bis 5 Mol-%, vorzugsweise von 0,005 bis 2 Mol-%, insbesondere von 0,01 bis 0,9 Mol-%, bezogen auf den Halogenaromaten der Formel (II) eingesetzt werden.

Bevorzugte Halogenaromaten der Formel (II) sind solche der Formel (II a), (II b), (II c) und (II d) 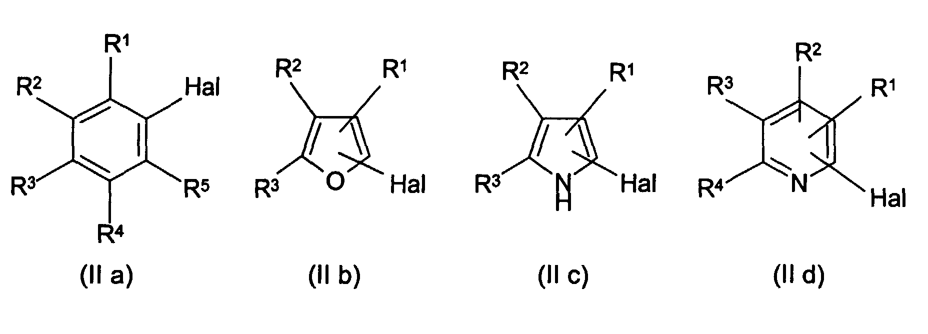 worin
Hal wie vorstehend definiert ist und
- R¹ bis R⁵: gleich oder verschieden sind und Wasserstoff, C₁-C₄-Alkyl, C₅-C₆-Cycloalkyl, C₁-C₄-Alkoxy, C₁-C₆-Acyloxy, Phenoxy, Phenyl, Fluor, Chlor, OH, NO₂, CN, COOH, NH Alkyl (C₁-C₄), N(C₁-C₄-alkyl)₂, NH₂, COO(C₁-C₄-alkyl), CO(C₁-C₄-alkyl), CF₃, SO₃H, SO₂R, wobei R Methyl, Ethyl oder Phenyl bedeutet,
bedeuten. Der Rest Hal kann dabei an jeder Position des aromatischen Ringes stehen.

Bevorzugte Amine der Formel (III) sind solche, worin
- R⁶ und R⁷: gleich oder verschieden sind und Wasserstoff, C₁-C₆-Alkyl, Hydroxyalkyl(C₁-C₆), Phenyl oder C₅-C₆-Cycloalkyl bedeuten, oder R⁶ und R⁷ bilden zusammen mit dem N-Atom einen Piperazin-, Piperidin-, Morpholin-, Imidazol-, Pyrazol- oder Pyrrolidin-Ring.

Mit dem erfindungsgemäßen Verfahren lassen sich beispielsweise Verbindungen wie Arylpiperazine, Arylpiperidine, Aryldibutylamine, Arylmorpholine, Arylphenylmethylamine, Aryldiethylamine, Aryldiphenylamine, wobei Aryl bevorzugt für Phenyl, Methoxyphenyl, Trifluormethylphenyl, Acetylphenyl, Fluorphenyl, Difluorphenyl, Chlorphenyl, Methylphenyl, Pyridyl, und Naphthyl steht, auf einfache Weise herstellen.

Zweckmäßigerweise wird das Amin der Formel (III) in einer Menge 1 bis 1,3 Mol, vorzugsweise 1 bis 1,1 Mol, je Mol Halogenaromat der Formel (II) und je auszutauschendem Hal-Atom eingesetzt.

Als Lösungsmittel finden im allgemeinen inerte organische Lösungsmittel Verwendung. Gut geeignet sind aromatische Kohlenwasserstoffe wie Toluol, Xylole, Anisol, Tetralin, und aliphatische Ether wie Tetrahydrofuran, Dimethoxyethan, Ethylenglykoldimethylether, Dioxan, Tetrahydropyran und Formaldehydacetale.

Die Reaktion läuft bei Temperaturen von 20 bis 200°C ab, vorzugsweise bei Temperaturen von 80 bis 180°C, insbesondere 100 bis 150°C.

Die Amine reagieren nach dem erfindungsgemäßen Verfahren mit Halogenaromaten bevorzugt in Gegenwart einer starken Base, deren pKa-Wert vorzugsweise >10 ist. Als Basen sind beispielsweise Alkali- oder Erdalkalialkoholate, Alkali- oder Erdalkaliamide sowie Butyllithium oder Phenyllithium anwendbar. Besonders bevorzugte Basen sind Alkali- und Erdalkalialkoholate wie Natrium-tert.-butanolat, Kalium-tert.-butanolat, Lithium-tert.-butanolat, Natrium-phenolat, Kaliumphenolat und Kaliumcarbonat, Natriumhexamethyldisilazid sowie Lithiumhexamethyldisilazid. Ganz besonders bevorzugt sind Natrium-tert.-butanolat, Kalium-tert.-butanolat und Lithium-tert.-butanolat.

Die Base wird vorzugsweise in einer Menge von 0,5 bis 5 Äquivalenten, insbesondere von 0,8 bis 3 Äquivalenten und ganz besonders bevorzugt von 1 bis 2, Äquivalenten bezogen auf den eingesetzten Halogenaromaten, eingesetzt.

Die nachstehenden Beispiele dienen zur Erläuterung des erfindungsgemäßen Verfahrens, ohne es darauf zu beschränken.

### Synthese des Katalysators

### trans-Di-µ-acetato-bis[2-[bis(1,1-dimethylethyl)phosphino]-2-methylpropyl-C,P]dipalladium (II)

5,10 mg (22,7 mmol) Pd(OAc)₂ werden in 200 ml Toluol gelöst. Die Lösung wird mit 5,00 mg (24,7 mmol) Tri-(tert.-butyl)phosphan versetzt. Die sich rasch nach hellorange aufklarende Lösung wird 10 Minuten lang auf 70-80°C erhitzt und dann auf Raumtemperatur abgekühlt. Das Lösungsmittel wird im Vakuum entfernt. Nach Zugabe von 200 ml Hexan kristallisiert nach kurzer Zeit das Produkt, trans-Di-µ-acetato-bis[2-[bis(1,1-dimethylethyl)phosphino]-2-methylpropyl-C,P]dipalladium, aus und wird abfiltriert. Man erhält einen weißgelben Feststoff (Schmp. > 200°C). Durch Umkristallisation aus Hexan und Filtration der Lösungen über Celite® kann das Produkt in Form weißgelber Kristallnadeln analysenrein gewonnen werden.

### Beispiel 1: Herstellung von 4-(N,N-Diethylamino)toluol

20 mmol p-Chlortoluol (2,53 g), 24 mmol Diethylamin (1,75 g), 28 mmol KOtBu (3,13 g) und 44 mg trans-Di-µ-acetato-bis[2-[bis(1,1-dimethylethyl)phosphino]-2-methylpropyl-C,P]dipalladium(II) (0,3 mol-%) werden in 50 ml Toluol suspendiert und 24 Stunden am Rückfluß gekocht. Nach dem Abkühlen auf Raumtemperatur filtriert man von den Salzen ab und wäscht mit Petrolether nach. Die Lösemittel werden am Rotationsverdampfer entfernt. Nach Kugelrohrdestillation (100°C/12 Torr) erhält man 4-(N,N-Diethylamino)toluol als farblose Flüssigkeit in einer Ausbeute von 87 %.

### Beispiel 2: Herstellung von 4-(N,N-Diethylamino)toluol in Gegenwart von LiBr

20 mmol p-Chlortoluol (2,53 g), 24 mmol Diethylamin (1,75 g), 28 mmol KOtBu (3,13 g), 4 mmol Lithiumbromid (0,35 g) und 44 mg trans-Di-µ-acetato-bis[2-[bis(1,1-dimethylethyl)phosphino]-2-methylpropyl-C,P]dipalladium(II) (0,3 mol-%) werden in 50 ml Toluol suspendiert und 24 Stunden am Rückfluß gekocht. Nach dem Abkühlen auf Raumtemperatur filtriert man von den Salzen ab und wäscht mit Petrolether nach. Die Lösemittel werden am Rotationsverdampfer entfernt. Nach Kugelrohrdestillation (100°C/12 Torr) erhält man 4-(N,N-Diethylamino)toluol als farblose Flüssigkeit in einer Ausbeute von 91 %.

### Beispiel 3: Herstellung von 2,5-Diphenyl-3-piperidinofuran

30 mmol 2,5-Diphenyl-3-chlorfuran (7,64 g), 32 mmol frisch destilliertes Piperidin (3,0 g), 32 mmol KOtBu (3,6 g), 2 mmol Lithiumchlorid (42,5 mg) und 44 mg trans-Di-µ-acetato-bis[2-[bis(1,1-dimethylethyl)phosphino]-2-methylpropyl-C,P]dipalladium(II) (0,2 mol-%) werden in 50 ml THF suspendiert und unter Schutzgas in ein Druckrohr gegeben. Nach 12 Stunden bei 110°C läßt man auf Raumtemperatur abkühlen, flitriert von den Salzen ab und wäscht mit Petrolether nach. Die Lösemittel werden am Rotationsverdampfer entfernt. Nach Kristallisation aus 96 %-igem Ethanol erhält man 2,5-Diphenyl-3-piperidinofuran als farblosen Feststoff; Ausbeute 84 %; Schmelzpunkt 103°C.

### Beispiel 4: Herstellung von N-Cyclohexylanilin

30 mmol Chlorbenzol (3,4 g), 31 mmol frisch destilliertes Cyclohexylamin (3,1 g), 31 mmol KOtBu (3,5 g), 20 mmol Lithiumchlorid (0,85 g) und 23 mg trans-Di-µ-chloro-bis[2-[bis(1,1-dimethylethyl)phosphino]-2-methylpropyl-C,P]dipalladium(II) (0,1 mol-%) werden in 50 ml Ethylenglykoldimethylether suspendiert und unter Schutzgas 9 Stunden am Rückfluß gekocht. Nach dem Abkühlen auf Raumtemperatur filtriert man von den Salzen ab und wäscht mit Petrolether nach. Die Lösemittel werden am Rotationsverdampfer im Vakuum entfernt. Nach Destillation erhält man N-Cyclohexylamin als farblose Flüssigkeit vom Siedepunkt 133°C/65 Torr, die bei Aufbewahrung im Kühlschrank langsam kristallisiert; Ausbeute 77 %.

### Beispiel 5: Herstellung von 1-(2,4-Dimethylphenyl)imidazol

30 mmol 2,4-Dimethylchlorbenzol (4,2 g), 40 mmol Imidazol (2,7 g), 35 mmol NaOEt-Lösung in Ethanol (10 Gew.-%) und 230 mg trans-Di-µ-chloro-bis[2-[bis(1,1-dimethylethyl)phosphino]-2-methylpropyl-C,P]dipalladium(II) (1,0 mol-%) werden in 50 ml THF suspendiert und in einem Druckrohr 9 Stunden auf 110°C erhitzt. Nach Filtration der Salze wird das Lösemittel im Vakuum entfernt. Man erhält rohes 1-(2,4-Dimethylphenyl)imidazol als braune Flüssigkeit, das durch Destillation (Siedepunkt 122°C/12 Torr) rein gewonnen werden kann; Ausbeute 48 %.

### Beispiel 6: Herstellung von N-(4-Chlorphenyl)piperidin

15 mmol p-Dichlorbenzol (2,2 g), 15,5 mmol Piperidin (1,3 g), 15,5 mmol NaOEt-Lösung in Ethanol (10 Gew.-%) und 50 mg trans-Di-µ-chloro-bis[2-[bis(1,1-dimethylethyl)-phosphino]-2-methylpropyl-C,P]dipalladium(II) werden in 75 ml Dioxan suspendiert und 15 Stunden am Rückfluß erhitzt. Nach wäßriger Aufarbeitung, Extraktion mit Petrolether und Trocknung über Natriumsulfat werden die Lösungsmittel im Vakuum entfernt. Man erhält rohes N-(4-Chlorphenyl)piperidin als hellbraune Flüssigkeit, die durch Destillation (Siedepunkt 50°C/0,08 Torr) gereinigt werden kann; Schmelzpunkt 69°C; Ausbeute 59 %.

### Beispiel 7: Herstellung von 1,4-Bis(N,N-dibutylamino)benzol

30 mmol p-Dichlorbenzol (4,4 g), 70 mmol Di-n-butylamin (9,0 g), 70 mmol KOtBu (7,9 g), 30 mmol Lithiumbromid (2,6 g) und 44 mg trans-Di-µ-acetato-bis[2-[bis(1,1-dimethylethyl)phosphino]-2-methylpropyl-C,P]dipalladium(II) (0,2 mol-%) werden in 75 ml Toluol suspendiert und unter Schutzgas in ein Druckrohr gegeben. Nach 12 Stunden bei 140°C arbeitet man wäßrig auf. Nach zweimaliger Extraktion der wäßrigen Phase mit Toluol werden die vereinigten organischen Phasen über Magnesiumsulfat getrocknet und am Rotationsverdampfer von den Lösungsmitteln befreit. Man erhält nach Destillation 1,4-Bis(N,N-dibutylamino)benzol als fast farblose Flüssigkeit vom Siedepunkt 143°C/10 Torr; Ausbeute 77 %.

## Patentansprüche

1. Verfahren zur Herstellung von aromatischen Aminen der Formel (I)
Ar-[NR⁶R⁷]ₙ (I)
worin n für die Zahl 1, 2 oder 3 steht,
Ar für unsubstituiertes oder substituiertes Phenyl, Furanyl, Pyrryl, Pyridinyl, Naphthyl oder Chinolinyl steht, worin die Substituenten 1, 2, 3, 4, 5 oder 6 an der Zahl und aus der Gruppe C₁-C₈-Alkyl, C₃-C₈-Cycloalkyl, Alkoxy-(C₁-C₈), Acyloxy-(C₁-C₈), C₆-C₁₀-Aryloxy, C₆-C₁₀-Aryl, Benzyl, Fluor, Chlor, Brom, OH, NO₂, OSO₂CF₃, CN, COOH, CHO, SO₃H, SO₂R, SOR, wobei R (C₁-C₄)-Alkyl, (C₆-C₁₀)-Aryl oder Benzyl bedeutet, NH₂, NHAlkyl (C₁-C₈), N-Alkyl₂-(C₁-C₈), CF₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, CHCH-CO₂Alkyl-(C₁-C₈), CHCHCO₂H, PO-Phenyl₂, PO-Alkyl₂-(C₁-C₄),
5-Ring-heteroaryl und 6-Ring-heteroaryl jeweils mit O, S und/oder N als Heteroatome sind; und
R⁶ und R⁷ unabhängig voneinander Wasserstoff, (C₁-C₁₂)-Alkyl, Hydroxyalkyl (C₁-C₁₂), unsubstituiertes oder substiutiertes Phenyl, oder C₃-C₈-Cycloalkyl sind, oder R⁶ und R⁷ zusammen mit dem N-Atom einen 5- oder 6-gliedrigen aliphatischen oder aromatischen Ring bilden, der 1 oder 2 weitere Atome aus der Gruppe N, O und S als Heteroatome enthalten kann,
durch Umsetzung von Halogenaromaten der allgemeinen Formel (II)
Ar-Hal (II)
worin Hal die Bedeutung Cl, Br oder l besitzt,
mit einem Amin der allgemeinen Formel (III)
R⁶R⁷NH (III)
dadurch gekennzeichnet, daß man die Umsetzung in Gegenwart eines Palladaphosphacyclobutans der allgemeinen Formel (IV) 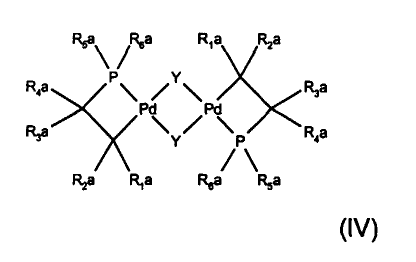 worin
R^{1a}, R^{2a} unabhängig voneinander Wasserstoff, (C₁-C₄)-Alkyl, (C₃-C₁₂)-Cycloalkyl, (C₁-C₄)-Alkoxy, Fluor, N-(C₁-C₄-Alkyl)₂, CO₂-(C₁-C₄-Alkyl), OCO-(C₁-C₄-Alkyl), oder substituiertes oder unsubstituiertes Aryl,
R^{3a}, R^{4a}, R^{5a} und R^{6a} unabhängig voneinander (C₁-C₈)-Alkyl, (C₃-C₁₂)-Cycloalkyl, substituiertes oder unsubstituiertes Aryl;
oder worin R^{1a} und R^{2a}, oder R^{2a} und R^{3a}, oder R^{3a} und R^{4a} zusammen einen aliphatischen Ring mit 4 bis 10 C-Atomen bilden,
oder worin R^{5a} und R^{6a} zusammen mit dem P-Atom, einen gesättigten oder ungesättigten 4- bis 9-gliedrigen Ring bilden, oder R^{4a} und R^{5a} eine überbrückende 1,ω-Alkandiyl-Kette mit 2 bis 7 C-Atomen bilden, und
Y ein Anion einer anorganischen oder organischen Säure, eine α,γ-Diketoverbindung oder einen 5- bis 6-gliedrigen stickstoffhaltigen Heterocyclus bedeutet,
in Gegenwart einer Base, und gegebenenfalls in Gegenwart eines ionischen Halogenides, in einem Lösemittel bei Temperaturen von 20 bis 200°C umsetzt.

2. Verfahren nach Anspruch 1, dadurch gekennzeichnet, daß
R^{1a} und R^{2a} unabhängig voneinander Wasserstoff, Methyl, Ethyl, Cyclopentyl, Cyclohexyl, Methoxy, Ethoxy, Fluor, Phenyl, Tolyl oder Naphthyl;
R^{3a} und R^{4a} unabhängig voneinander (C₁-C₄)-Alkyl, (C₅-C₆)-Cycloalkyl, substituiertes oder unsubstituiertes C₆-C₁₀-Aryl oder worin R^{3a} und R^{4a} zusammen einen aliphatischen Ring mit 5 bis 6 C-Atomen bilden;
R^{5a} und R^{6a} unabhängig voneinander (C₁-C₄)-Alkyl, (C₅-C₆)-Cycloalkyl, Phenyl, Naphthyl, Anthracenyl, die unsubstituiert oder mit 1 bis 3 CF₃-, (C₁-C₄)-Alkyl oder (C₁-C₄)-Alkoxy Gruppen substituiert sind;
und Y für Acetat, Propionat, Benzoat, Chlorid, Bromid, Iodid, Fluorid, Sulfat, Hydrogensulfat, Nitrat, Phosphat, Trifluoromethansulfonat,Tetrafluoroborat, Tosylat, Mesylat, Acetylacetonat, Hexafluoracetylacetonat oder Pyrazolyl steht, bedeuten.

3. Verfahren nach Anspruch 1 oder 2, dadurch gekennzeichnet, daß
R^{1a} und R^{2a} unabhängig voneinander Wasserstoff oder Methyl;
R^{3a} und R^{4a} unabhängig voneinander Methyl, Ethyl oder Phenyl;
R^{5a} und R^{6a} unabhängig voneinander Phenyl, Naphthyl, o-Trifluoromethylphenyl, o-Trifluoromethyl-p-tolyl, o-Trifluoromethyl-p-methoxy-phenyl, o-Methoxy-phenyl, o,p-Dimethoxy-phenyl, o,o,p-Trimethoxyphenyl, Anthracenyl, tert.-Butyl, n-Butyl, Isopropyl, Isobutyl, Cyclohexyl oder 1-Methylcyclohexyl bedeuten.

4. Verfahren nach mindestens einem der Ansprüche 1 bis 3, dadurch gekennzeichnet, daß die Verbindung der Formel (IV) trans-Di-µ-acetato-bis[2-[bis(1,1-dimethylethyl)phosphino]-2-methylpropyl-C,P]dipalladium (II), trans-Di-µ-acetato-bis[2-[(1,1-dimethylethyl)-phenylphosphino]-2-methylpropyl-C,P]dipalladium (II), trans-Di-µ-chloro-bis[2-[bis(1,1-dimethylethyl)phosphino]-2-methylpropyl-C,P]dipalladium (II), trans-Di-µ-chloro-bis[2-[(1,1-dimethylethyl)-phenylphosphino]-2-methylpropyl-C,P]dipalladium (II), trans-Di-µ-bromo-bis[2-[bis(1,1-dimethylethyl)phosphino]-2-methylpropyl-C,P]dipalladium (II) oder trans-Di-µ-bromo-bis[2-[(1,1-dimethylethyl)-phenylphosphino]-2-methylpropyl-C,P]dipalladium (II) ist.

5. Verfahren nach mindestens einem der Ansprüche 1 bis 4, dadurch gekennzeichnet, daß die Verbindung der Formel (IV) in Substanz zugegeben wird.

6. Verfahren nach mindestens einem der Ansprüche 1 bis 5, dadurch gekennzeichnet, daß die Verbindung der Formel (IV) in einer Menge von 0,001 bis 5 Mol-%, insbesondere 0,01 bis 0,9 Mol-%, bezogen auf den Halogenaromaten der Formel (II), eingesetzt wird.

7. Verfahren nach mindestens einem der Ansprüche 1 bis 6, dadurch gekennzeichnet, daß der Halogenaromat die Formel (IIa), (IIb), (IIc), oder (IId) hat 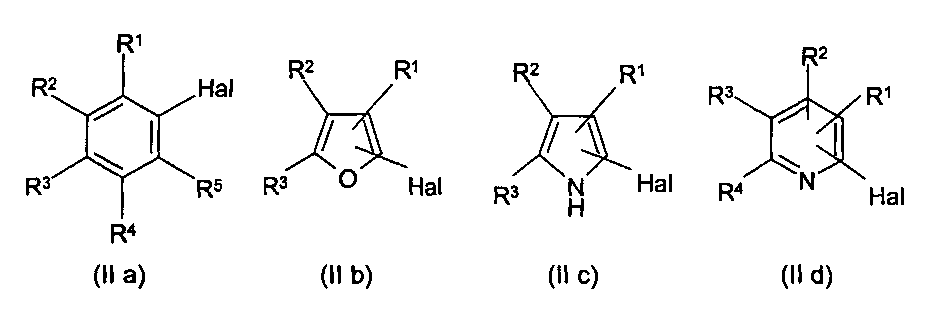 worin
R¹ bis R⁵ gleich oder verschieden sind und Wasserstoff, C₁-C₄-Alkyl, C₅-C₆-Cycloalkyl, C₁-C₄-Alkoxy, C₁-C₆-Acyloxy, Phenoxy, Phenyl, Fluor, Chlor, OH, NO₂, ON, COOH, NH Alkyl (C₁-C₄), N(C₁-C₄-alkyl)₂, COO(C₁-C₄-alkyl), CO(C₁-C₄-alkyl), CF₃, SO₃H, SO₂R, wobei R Methyl, Ethyl oder Phenyl bedeutet,
bedeuten.

8. Verfahren nach mindestens einem der Ansprüche 1 bis 7, dadurch gekennzeichnet, daß R⁶ und R⁷ gleich oder verschieden sind und Wasserstoff, C₁-C₆-Alkyl, Hydroxyalkyl(C₁-C₆), Phenyl oder C₅-C₆-Cycloalkyl bedeuten, oder R⁶ und R⁷ bilden zusammen mit dem N-Atom einen Piperazin-, Piperidin-, Morpholin-, Imidazol-, Pyrazol- oder Pyrrolidin-Ring.

9. Verfahren nach mindestens einem der Ansprüche 1 bis 8, dadurch gekennzeichnet, daß das ionische Halogenid ein Alkali-, Ammonium-, Alkylammonium-, Alkylolammonium- oder Phosphoniumhalogenid ist, wobei Halogenid die Bedeutung Chlorid, Bromid oder Jodid hat.

10. Verfahren nach mindestens einem der Ansprüche 1 bis 9, dadurch gekennzeichnet, daß die Base ein Alkali- oder Erdalkalialkoholat, Alkali- oder Erdalkaliamid, Butyllithium, Phenyllithium, Lithium- oder Natriumhexamethyldisilazid ist.
